(19) Europäisches Patentamt
European Patent Office
Office européen des brevets

(11) Veröffentlichungsnummer: **0 107 161**
**B1**

# (12) EUROPÄISCHE PATENTSCHRIFT

(45) Veröffentlichungstag der Patentschrift: **09.08.89**

(51) Int. Cl.⁴: **A 23 L 1/30**, A 23 K 1/16, A 61 K 31/70

(21) Anmeldenummer: **83110325.4**

(22) Anmeldetag: **17.10.83**

## (54) Mittel und Verfahren zur Optimierung der Gewebemasse von Organen innerhalb der genetischen Schwankungsbreite bei Menschen und Tieren.

(30) Priorität: **26.10.82 CH 6243/82**
**15.11.82 CH 6652/82**

(43) Veröffentlichungstag der Anmeldung:
**02.05.84 Patentblatt 84/18**

(45) Bekanntmachung des Hinweises auf die Patenterteilung: **09.08.89 Patentblatt 89/32**

(84) Benannte Vertragsstaaten:
**AT BE CH DE FR GB IT LI LU NL SE**

(56) Entgegenhaltungen:
**DE–A– 1 470 190**
**DE–B– 1 212 826**
**FR–A– 1 392 643**
**FR–A– 1 502 955**
**FR–A– 2 004 476**
**FR–A– 2 131 249**
**GB–A– 1 046 770**
**US–A– 2 328 355**
**CHEMICAL ABSTRACTS, Band 92, 1980, Seite 178, Zusammenfassung Nr. 175821v, COLUMBUS, Ohio (US)**
**Collaborative Research Inc., Catalogue (1982) Products for Biotechnology, pages 25-26**
**Die Akte enthält technische Angaben, die nach dem Eingang der Anmeldung eingereicht wurden und die nicht in dieser Patentschrift enthalten sind.**

(73) Patentinhaber: **CTA Finanz AG**
**Guggiweg 8**
**CH-6300 Zug (CH)**

(72) Erfinder: **Moor, Hermann**
**Rheinstrasse 28**
**CH-4302 Augst (CH)**

(74) Vertreter: **Becher, Pauline, Dr. et al**
**A. Braun, Braun, Héritier, Eschmann AG Holbeinstrasse 36-38**
**CH-4051 Basel (CH)**

**EP 0 107 161 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Aus dem Merck Index, 9. Ausgabe, Rahway, N. Y., USA 1976 ist bekannt, dass Orotsäure, d. h. eine Uracilcarbonsäure der Formel :

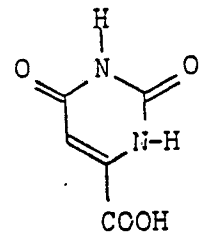

als Futterzusatz in Kombination mit Methionin zur Förderung des Wachstums von Kälbern vorgeschlagen worden ist. In der CH-PS Nr. 505.560 wird vorgeschlagen, zur Verbesserung des Wachstums und weiterer nützlicher Eigenschaften von Nutztieren dem Futter eine Thiouracilcarbonsäure, deren nichttoxisches Salz und/oder deren Alkylester zuzusetzen. Gewünschtenfalls kann man ausserdem ein biologisches Methylierungsmittel, wie Methionin, und/oder eine Uracilcarbonsäure, deren nichttoxisches Salz und/ oder deren Alkylester zugeben.

Aus der FR-A-1 502 955 ist die Verwendung von 5'-Pyrimidin-Mononucleotiden zur Förderung des Wachstums von Menschen und Tieren bekannt. In diesem Dokument finden sich aber nur Zahlenangaben über die Zunahme des Gesamtkörpergewichts von Kücken und Schweinen bei Verabreichung dieser Wirkstoffe im Futter, also über eine unspezifische wachstumsfördernde Wirkung. Von irgendeiner selektiven Wirkung auf bestimmte Organe ist nirgends die Rede.

Aus der US-A-2 328 355 ist ein Mittel zur Förderung des Wachstums von Tieren bekannt, das Uracilcarbonsäuren als Wirkstoffe enthält. Es ist aber aus diesem Dokument nur zu entnehmen, dass die Wirkstoffe das Gesamtgewicht der Versuchstiere erhöhen, also ebenfalls nur eine unspezifische wachstumsfördernde Wirkung haben. Auch hier finden sich keinerlei Hinweise auf eine selektive Wirkung auf bestimmte Organe.

Es wurde nun gefunden, dass bestimmte Oligonucleotide, Nucleinsäurebasen und Carboxylderivate sowie Aminoderivate von Nucleinsäurebasen dazu verwendet werden können, die Gewebemasse von Organen bei Menschen und Tieren innerhalb ihrer genetischen Schwankungsbreite zu optimieren. Bekanntlich kann die genetische Information, die in der DNS von Menschen und Tieren verschlüsselt ist, bei der Proteinbiosynthese in unterschiedlichem Ausmass realisiert werden, wodurch sich eine gewisse Schwankungsbreite hinsichtlich der Ausbildung der entsprechenden Organe ergibt. Die oben genannten Wirkstoffe ermöglichen es nun, die genetischen Informationen optimal zu realisieren.

Es hat sich ferner gezeigt, dass Adenin sowie seine Carboxylderivate und Aminoderivate und Oligonucleotide, die als endständige Basen Adenin enthalten, für die Erfindung nicht brauchbar sind. Die Erfindung bezieht sich somit auf ein Mittel zur organspezifischen Optimierung der Gewebemasse von Organen innerhalb der genetischen Schwankungsbreite bei Menschen und Tieren, das dadurch gekennzeichnet ist, dass es

a) mindestens eine Nucleinsäurebase mit Ausnahme von Adenin und/oder
b) mindestens ein Oligonucleotid, dessen endständige Basen von Adenin verschieden sind, als Wirkstoff(e) in Kombination mit einem Träger mit Ausnahme von Wasser enthält .

Ferner bezieht sich die Erfindung auf ein Verfahren zur nicht-therapeutischen, organspezifischen Optimierung der Gewebemasse von Organen innerhalb der genetischen Schwankungsbreite bei Tieren, das dadurch gekennzeichnet ist, dass man

a) mindestens eine Nucleinsäurebase mit Ausnahme von Adenin und/oder
b) mindestens ein Oligonucleotid, dessen endständige Basen von Adenin verschieden sind,

als Wirkstoff(e) in den tierischen Körper einbringt.

Unter « Oligonucleotiden » versteht man bekanntlich lineare Sequenzen aus maximal 20 Bausteinen, die je nach Kettenlänge als Di-, Tri-, Tetra-, Penta-nucleotide usw. bezeichnet werden. Bei den Oligonucleotiden sind die endständigen Zucker bekanntlich phosphoryliert.

Die Wirkung der oben genannten Wirkstoffe kann man dadurch erklären, dass sie in die Zellen eindringen und dort die lytischen Enzyme, vor allem die Desaminasen, hemmen. Dadurch können diese Emzyme keine oder jedenfalls weniger Aminosäuren, die aus den verdauten Proteinen stammen, zerstören. Infolgedessen stehen für den Synthesestoffwechsel mehr Aminosäuren zur Verfügung, was zu einer schnelleren Vermehrung und einer verbesserten Differenzierung der betreffenden Zellen führt.

Im Verdauungstrakt von Mensch und Tier sind Pankreas-Ribonucleasen vorhanden, die Ribonucleinsäure zu Nucleotiden abbauen. Man könnte daher annehmen, dass durch Verabreichung von Ribonucleinsäure aus Hefe oder anderen Quellen die gleichen Wirkungen erzielt werden könnten wie mit den erfindungsgemäss verwendeten Wirkstoffen, da die Ribonucleinsäuren ja auch zu Nucleotiden abgebaut werden. Ueberraschenderweise hat sich aber gezeigt, dass dies nicht der Fall ist.

Die oben genannten Wirkstoffe beeinflussen generell Zellverbände, die sich stark teilen bzw. die schnell wachsen. Wenn die Wirkstoffe den Muttertieren vor der Geburt und/oder den Jungtieren unmittelbar nach der Geburt verabreicht werden, bewirken sie eine intensive Entwicklung der innersekretorischen Drüsen und des lymphatischen Systems im Embryonalzustand bzw. kurz nach der Geburt. Die Wirkstoffe fördern auch das Wachstum von sich bewegenden Muskeln. Die Wirkung erstreckt sich aber je nach der Zusammensetzung der Wirkstoffe bevorzugt auf verschiedene Organe. Z. B. beeinflussen Nucleinsäurebasen und Di- bis Tetranucleotide besonders das embryonale Gewebe, während Octabis Tetradecanucleotide besonders auf bewegte Muskeln einwirken. Der Fachmann kann durch Versuche ohne weiteres feststellen, auf welche Gewebe bestimmte Wirkstoffe eine besonders starke Wirkung ausüben.

Wenn die Entwicklung bestimmter Organe durch die obigen Wirkstoffe im Embryonalzustand gefördert worden ist, ergeben sich die folgenden Wirkungen :

Eine bessere Entwicklung der Hypophyse führt zu einer höheren Produktion von somatotropem Hormon, dem natürlichen Wachstumshormon.

Eine bessere Entwicklung der Sexualorgane bewirkt bessere Zuchtresultate.

Eine bessere Entwicklung des Pankreasgewebes verursacht eine gesteigerte Insulinsekretion, die ein schnelleres Wachstum begünstigt.

Eine bessere Entwicklung von lymphatischem System und Thymus ergibt eine erhöhte Resistenz gegen Krankheiten.

Es wurde z. B. gefunden, dass es bei Verabreichung eines Gemisches von Nucleinsäurebasen mit Di-, Tri- und eventuell Tetranucleotiden an BALB/c-Mäuse oder Swiss-Mäuse zu einem statistisch signifikanten Anstieg der Antikörper produzierenden Milzzellen kommt.

Die gleichen Wirkstoffe ergeben bei der Untersuchung der Beeinflussbarkeit der komplementabhängigen Immunhämolyse in vitro unter Verwendung von Humankomplement bereits in geringer Konzentration eine starke Komplementaktivierung.

Die erfindungsgemäss verwendeten Wirkstoffe sind bekannt und können in an sich bekannter Weise hergestellt werden.

Die erfindungsgemässen Mittel bzw. die Wirkstoffe können durch Infusion oder Injektion oder per os verabreicht werden. Als Träger für die erfindungsgemässen Wirkstoffe eignen sich je nach Art der Verabreichung die bekannten, pharmazeutisch bzw. veterinär unbedenklichen Träger mit Ausnahme von Wasser oder bei Tieren auch das Futter. Beispiele von geeigneten Trägern sind Lactose, Kartoffelund Maisstärke, Talk, Gelatine, Stearinsäure, Kieselsäure, sterile Flüssigkeiten mit Ausnahme von Wasser oder Oele, z. B. Erdnussöl.

Die erfindungsgemässen Mittel können als Konzentrate oder Dosis-Einheiten, wie Kapseln, Tabletten oder Dragees vorliegen. In diesem Falle enthalten sie in der Regel 1 bis 200 mg, vorzugsweise 30 bis 150 mg, insbesondere ca. 80 mg Wirkstoff(e) pro g. Wenn sie hingegen als gebrauchsfertiges Futtermittel vorliegen, enthalten sie normalerweise 1 bis 200 mg, vorzugsweise 30 bis 150 mg, insbesondere ca. 80 mg Wirkstoff(e) pro kg. Gewöhnlich verabreicht man die Wirkstoffe in Mengen von 0,02 bis 10 mg, vorzugsweise 2 bis 6 mg insbesondere ca. 3 mg pro kg Körpergewicht und Tag.

Herstellung der Wirkstoffe

Die Nucleinsäurebasen und Oligonucleotide wurden nach den Angaben in die Hefen, Band 1 : Die Hefen in der Wissenschaft, Verlag Hans Carl, Nürnberg 1960 (S. 415 ff, S. 435 ff und S. 439 sowie Literaturverzeichnis auf S. 440 bis 445) hergestellt und getrennt. In den Beispielen wurde eine niedermolekulare Fraktion (NF) bzw. eine höhermolekulare Fraktion (HF) aus der enzymatischen Spaltung von Hefe-RNS verwendet. Die Mengenverhältnisse der einzelnen Bausteine wurden nicht verändert und waren gleich wie in der als Ausgangsmaterial verwendeten RNS. Die NF bestand aus Nucleinsäurebasen sowie Mono-, Di-, Tri- und Tetranucleotiden. Die HF enthielt die höheren Oligonucleotide (Octa- bis Tetradecanucleotide).

Beispiel 1

80 g HF-Trockensubstanz wurden mit je 25 g der Nucleinsäurebasen Uracil und Guanin sowie 870 g Molkenpulver homogen vermischt. 500 g dieses Gemisches wurden in 1 000 kg eines Ferkelmast-Alleinfutters eingemischt. Mit dieser Futtermischung wurden Ferkel von 25 kg Lebendgewicht bis zum Endmastgewicht von 100 kg ernährt.

Bei den Ferkeln, die mit dem das erfindungsgemässe Mittel enthaltenden Futter gefüttert worden waren, war der Musculus longissimus dorsi (das Karree) viel stärker entwickelt als bei Ferkeln, die mit dem gleichen Futter ohne Zusatz des erfindungsgemässen Mittels bis zum gleichen Endmastgewicht gefüttert worden waren. Die in Fachkreisen übliche Methode zur Erfassung dieses Effektes besteht in der

Messung der sogenannten Karreefläche, d. h. des Querschnittes des Musculus longissimus dorsi. Im vorliegenden Beispiel wurde eine statistisch gesicherte Vergrösserung der Karreefläche von 31,8 $cm^2$ auf 35,5 $cm^2$ erzielt.

Beispiel 2

100 g NF-Trockensubstanz wurden mit 45 g der Nucleinsäurebase Uracil und 40 g Uracilcarbonsäure sowie 815 g « Distillers' dried solubles » als Träger homogen vermischt. 500 g dieses Konzentrates wurden 1 000 kg eines in allen nutritiven Hinsichten vollwertigen Zuchtschweine-Alleinfutters beigemischt. Damit wurden trächtige Zuchtsauen bis zum Werfen ernährt.

Die gesamte Drüsenmasse der Ferkel, bestehend aus Thymus, Lymphknoten, Hypophyse, Nebenniere, Pankreas und Leber, war um 15 bis 25 % erhöht, d. h. bis zur Obergrenze der genetischen Schwankungsbreite. Statt Uracilcarbonsäure kann man auch Aminouracil verwenden.

Beispiel 3

Je 150 mg HF-Trockensubstanz wurden in magensaftresistente Gelatinekapseln gefüllt. Je eine Kapsel wurde dreimal täglich an erwachsene Menschen verabreicht, die an einem « Body-Building »-Training teilnahmen. Die Gewebemasse (das Volumen) aller bewegten Kraftmuskeln war bei sonst gleicher Ernährung, Bewegung und Belastung deutlich sichtbar und gut messbar vergrössert. Dies drückte sich auch in einer objektiv messbaren Leistungssteigerung aus.

**Patentansprüche**

1. Mittel zur organspezifischen Optimierung der Gewebemasse von Organen innerhalb der genetischen Schwankungsbreite bei Menschen und Tieren, dadurch gekennzeichnet, dass es
   a) mindestens eine Nucleinsäurebase mit Ausnahme von Adenin und/oder
   b) mindestens ein Oligonucleotid, dessen endständige Basen von Adenin verschieden sind,
als Wirkstoff(e) in Kombination mit einem Träger mit Ausnahme von Wasser enthält.

2. Mittel nach Anspruch 1, dadurch gekennzeichnet, dass es als Wirkstoff(e) mindestens ein Di- bis Tetranucleotid, dessen endständige Basen von Adenin verschieden sind, sowie gegebenenfalls mindestens eine Nucleinsäurebase mit Ausnahme von Adenin und/oder mindestens ein Carboxylderivat einer Nucleinsäurebase mit Ausnahme von Adenin und/oder mindestens ein Aminoderivat einer Nucleinsäurebase mit Ausnahme von Adenin enthält.

3. Mittel nach Anspruch 1, dadurch gekennzeichnet, dass es als Wirkstoff(e) mindestens ein Octa- bis Tetradecanucleotid, dessen endständige Basen von Adenin verschieden sind, sowie gegebenenfalls mindestens eine Nucleinsäurebase mit Ausnahme von Adenin und/oder mindestens ein Carboxylderivat einer Nucleinsäurebase mit Ausnahme von Adenin und/oder mindestens ein Aminoderivat einer Nucleinsäurebase mit Ausnahme von Adenin enthält.

4. Mittel nach einem der Ansprüche 1 bis 3 in Form eines Konzentrats oder einer Dosis-Einheit, dadurch gekennzeichnet, dass es 1 bis 200 mg, vorzugsweise 30 bis 150 mg, insbesondere ca. 80 mg Wirkstoff(e) pro g enthält.

5. Mittel nach einem der Ansprüche 1 bis 3 in Form eines gebrauchsfertigen Futtermittels, dadurch gekennzeichnet, dass es 1 bis 200 mg, vorzugsweise 30 bis 150 mg, insbesondere ca. 80 mg Wirkstoff(e) pro kg enthält.

6. Verfahren zur nicht-therapeutischen, organspezifischen Optimierung der Gewebemasse von Organen innerhalb der genetischen Schwankungsbreite bei Tieren, dadurch gekennzeichnet, dass man
   a) mindestens eine Nucleinsäurebase mit Ausnahme von Adenin und/oder
   b) mindestens ein Oligonucleotid, dessen endständige Basen von Adenin verschieden sind,
als Wirkstoff(e) in den tierischen Körper einbringt.

7. Verfahren nach Anspruch 6, dadurch gekennzeichnet, dass man als Wirkstoff(e) mindestens ein Di- bis Tetranucleotid, dessen endständige Basen von Adenin verschieden sind, sowie gegebenenfalls mindestens eine Nucleinsäurebase mit Ausnahme von Adenin und/oder mindestens ein Carboxylderivat einer Nucleinsäurebase mit Ausnahme von Adenin und/oder mindestens ein Aminoderivat einer Nucleinsäurebase mit Ausnahme von Adenin verwendet, um speziell das Wachstum von embryonalem Gewebe zu fördern.

8. Verfahren nach Anspruch 6, dadurch gekennzeichnet, dass man als Wirkstoff(e) mindestens ein Octabis Tetradecanucleotid, dessen endständige Basen von Adenin verschieden sind, sowie gegebenenfalls mindestens eine Nucleinsäurebase mit Ausnahme von Adenin und/oder mindestens ein Carboxylderivat einer Nucleinsäurebase mit Ausnahme von Adenin und/oder mindestens ein Aminoderivat einer Nucleinsäurebase mit Ausnahme von Adenin verwendet, um speziell das Wachstum von bewegten Muskeln zu fördern.

9. Verfahren nach Anspruch 6, dadurch gekennzeichnet, dass man den Wirkstoff bzw. die Wirkstoffe einem trächtigen Tier oder kurz nach der Geburt einem Jungtier verabreicht, um speziell die innersekretorischen Drüsen und das lymphatische System intensiv zu entwickeln.

10. Verfahren nach einem der Ansprüche 6 bis 9, dadurch gekennzeichnet, dass man den Wirkstoff bzw. die Wirkstoffe durch Infusion oder Injektion oder per os, mit dem Futter, verabreicht.

11. Verfahren nach einem der Ansprüche 6 bis 10, dadurch gekennzeichnet, dass man 0,02 bis 10 mg, vorzugsweise 2 bis 6 mg, insbesondere ca. 3 mg Wirkstoff(e) pro kg Körpergewicht verabreicht.

**Claims**

1. Means for organ-specific optimisation of the tissue mass of organs within the genetic fluctuation range in humans and animals, characterised in that it contains
   a) at least one nucleic acid base with the exception of adenine and/or
   b) at least one oligonucleotide, the terminal bases of which are different from adenine,

as the active substance(s) in conjunction with a carrier, with the exception of water.

2. Means according to claim 1, characterised in that it contains as active substance(s) at least one di- to tetranucleotide, the terminal bases of which are different from adenine, optionally together with at least one nucleic acid base with the exception of adenine and/or at least one carboxyl derivative of a nucleic acid base with the exception of adenine and/or at least one amino derivative of a nucleic acid base with the exception of adenine.

3. Means according to claim 1, characterised in that it contains as active substance(s) at least one octa- to tetradecanucleotide, the terminal bases of which are different from adenine, optionally together with at least one nucleic acid base with the exception of adenine and/or at least one carboxyl derivative of a nucleic acid base with the exception of adenine and/or at least one amino derivative of a nucleic acid base with the exception of adenine.

4. Means according to any one of claims 1 to 3 in the form of a concentrate or a dosage unit, characterised in that it contains 1 to 200 mg, preferably 30 to 150 mg, more particularly about 80 mg of active substance(s) per gram.

5. Means according to any one of claims 1 to 3 in the form of a feedstuff ready for use, characterised in that it contains 1 to 200 mg, preferably 30 to 150 mg, more particularly about 80 mg of active substance(s) per kg.

6. Process for the non-therapeutic, organ-specific optimisation of the tissue mass of organs within the genetic fluctuation range in animals, characterised in that
   a) at least one nucleic acid base with the exception of adenine and/or
   b) at least one oligonucleotide, the terminal bases of which are different from adenine,

is or are introduced as active substance(s) into the animal's body.

7. Process according to claim 6, characterised in that the active substance or substances used is or are at least one di- to tetranucleotide, the terminal bases of which are different from adenine, optionally together with at least one nucleic acid base with the exception of adenine and/or at least one carboxyl derivative of a nucleic acid base with the exception of adenine and/or at least one amino derivative of a nucleic acid base with the exception of adenine, in order to promote in particular the growth of embryonic tissue.

8. Process according to claim 6, characterised in that the active substance or substances used is or are at least one octa- to tetradecanucleotide, the terminal bases of which are different from adenine, optionally together with at least one nucleic acid base with the exception of adenine and/or at least one carboxyl derivative of a nucleic acid base with the exception of adenine and/or at least one amino derivative of a nucleic acid base with the exception of adenine, in order to promote in particular the growth of moving muscles.

9. Process according to claim 6, characterised in that the active substance or substances is or are administered to a pregnant animal or to a young animal shortly after birth, in order, in particular, to develop intensively the internal secretory glands and the lymphatic system.

10. Process according to any one of claims 6 to 9, characterised in that the active substance or substances is or are administered by infusion or injection or by oral route, with the feed.

11. Process according to any one of claims 6 to 10, characterised in that 0.02 to 10 mg, preferably 2 to 6 mg, more particularly about 3 mg of active substance or substance(s) are administered per kg of body weight.

**Revendications**

1 Agent pour l'optimalisation organo-spécifique de la masse de tissu d'organes dans le domaine de la plage génétique chez l'homme et les animaux caractérisé en ce qu'il contient
   a) au moins une base d'acide nucléique à l'exception de l'adénine et/ou
   b) au moins un oligonucléotide dont les bases terminales sont différentes de l'adénine,

comme principe(s) actif(s) en combinaison avec un excipient, à l'exception de l'eau.

2. Agent suivant la revendication 1, caractérisé en ce qu'il contient comme principe(s) actif(s) au

moins un di- à tétranucléotide dont les bases terminales sont différentes de l'adénine, ainsi qu'éventuellement au moins une base d'acide nucléique à l'exception de l'adénine et/ou au moins un dérivé carboxylé d'une base d'acide nucléique à l'exception de l'adénine et/ou au moins un dérivé aminé d'une base d'acide nucléique à l'exception de l'adénine.

3. Agent suivant la revendication 1, caractérisé en ce qu'il contient comme principe(s) actif(s) au moins un octa- à tétradécanucléotide dont les bases terminales sont différentes de l'adénine ainsi qu'éventuellement au moins une base d'acide nucléique à l'exception de l'adénine et/ou au moins un dérivé carboxylé d'une base d'acide nucléique à l'exception de l'adénine et/ou au moins un dérivé aminé d'une base d'acide nucléique à l'exception de l'adénine.

4. Agent suivant l'une quelconque des revendications 1 à 3 sous la forme d'un concentré ou d'une dose unitaire, caractérisé en ce qu'il contient 1 à 200 mg de préférence 30 à 150 mg et en particulier environ 80 mg de principe(s) actif(s).

5. Agent suivant l'une quelconque des revendications 1 à 3 sous la forme d'un aliment prêt à la consommation caractérisé en ce qu'il contient 1 à 200 mg de préférence 30 à 150 mg et en particulier environ 80 mg de principe(s) actif(s).

6. Procédé pour l'optimalisation organo-spécifique non thérapeutique de la masse de tissu d'organes dans le domaine de la plage génétique chez les animaux, caractérisé en ce qu'on introduit dans le corps des animaux comme principe(s) actif(s)

a) au moins une base d'acide nucléique à l'exception de l'adénine et/ou

b) au moins un oligonucléotide dont les bases terminales sont différentes de l'adénine.

7. Procédé suivant la revendication 6, caractérisé en ce qu'on utilise comme principe(s) actif(s) au moins un di- à tétranucléotide dont les bases terminales sont différentes de l'adénine, ainsi qu'éventuellement au moins une base d'acide nucléique à l'exception de l'adénine et/ou au moins un dérivé carboxylé d'une base d'acide nucléique à l'exception de l'adénine et/ou au moins un dérivé aminé d'une base d'acide nucléique à l'exception de l'adénine, pour accélérer spécialement la croissance des tissus embryonnaires.

8. Procédé suivant la revendication 6, caractérisé en ce qu'on utilise comme principe(s) actif(s) au moins un octa- à tétranucléotide dont les bases terminales sont différentes de l'adénine, ainsi qu'éventuellement au moins une base d'acide nucléique à l'exception de l'adénine et/ou au moins un dérivé carboxylé d'une base d'acide nucléique à l'exception de l'adénine et/ou au moins un dérivé aminé d'une base d'acide nucléique à l'exception de l'adénine, pour accélérer spécialement la croissance des muscles moteurs.

9. Procédé suivant la revendication 6, caractérisé en ce qu'on administre le principe actif ou les principes actifs à un animal gravide ou peu après la naissance à un jeune animal spécialement pour développer intensivement les glandes à sécrétion interne et le système lymphatique.

10. Procédé suivant l'une quelconque des revendications 6 à 9, caractérisé en ce qu'on administre le principe actif ou les principes actifs par perfusion ou injection ou bien per os avec les aliments.

11. Procédé suivant l'une quelconque des revendications 6 à 10, caractérisé en ce qu'on administre 0,02 à 10 mg, de préférence 2 à 6 mg et en particulier environ 3 mg de principe(s) actif(s) par kg de poids du corps.